# EUROPEAN PATENT SPECIFICATION

(11) **EP 1 908 419 B1**
(45) Date of publication and mention of the grant of the patent: **02.09.2009**
(21) Application number: 06021056.4
(22) Date of filing: 06.10.2006
(51) Int. Cl.: A61B 17/11, A61B 17/00, A61B 17/12

(54) **A locking device for an anastomotic device**
Verriegelungsvorrichtung für eine Anastomosevorrichtung
Dispositif de verrouillage pour un dispositif pour l'anastomose

(43) Date of publication of application: 09.04.2008
(73) Proprietor: ETHICON ENDO-SURGERY, INC., Cincinnati, OH 45242-2839 (US)
(72) Inventor: Thompson, Brian James, Cincinnati Ohio 45226 (US); Oritz, Mark S., Milford Ohio 45150 (US); Pastorelli, Alessandro, 00136 Roma (IT)
(74) Representative: Leihkauf, Steffen Falk

(56) References cited:
- EP-A- 1 520 530
- EP-A1- 0 517 488
- EP-A2- 0 119 848
- WO-A-98/03118
- WO-A-20/05110240
- WO-A-20/06102213
- US-A1- 2005 038 456
- US-B1- 6 682 541

## Description

The present invention relates, in general, to devices and methods for surgically modifying organs and vessels.

More particularly, it relates to anastomosis devices for joining two organs such as, for example, two separate lengths of small bowel to each other, a section of small bowel to the stomach, or the common bile duct to the duodenum in a procedure called a choledochoduodenostomy. Vascular anastomosis may be performed as well.

The percentage of the world population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and though temporarily effective, failed to correct the condition. Further, introducing an object in the stomach, such as an esophago-gastric balloon, to fill the stomach have also been used to treat the condition; however, such approaches tend to cause irritation to the stomach and are not effective long-term.

Surgical treatments of morbid obesity have been increasingly used with greater success. These approaches may be generalized as those that reduce the effective size of the stomach, limiting the amount of food intake, and those that create malabsorption of the food that is eaten. For instance, some patients benefit from adjustable gastric bands (AGB) that are laparoscopically placed about the stomach to form a stoma of a desired size that allows food to fill an upper portion of the stomach, causing a feeling of satiety. To allow adjustment of the size of the stoma after implantation, a fluid conduit communicates between an inwardly presented fluid bladder of the AGB to a fluid injection port subcutaneously placed in front of the patient's sternum. A syringe needle may then inject or withdraw fluid as desired to adjust the AGB.

Although an effective approach to obesity for some, other patients may find the lifestyle changes undesirable, necessitated by the restricted amount of food intake. In addition, the medical condition of the patient may suggest the need for a more permanent solution. To that end, surgical approaches have been used to alter the portions of the stomach and/or small intestine available for digesting food. Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

With particular reference to gastric bypass procedures, current methods of performing a laparoscopic anastomosis for a gastric bypass include stapling, suturing, and placing biofragmentable rings, each having significant challenges. For instance, suturing is time consuming, as well as being technique and dexterity dependent. Stapling requires placement of an anvil, which is a large device that cannot be introduced through a trocar port. Having to introduce the port through a laparotomy presents an increased incidence of wound site infection associated with intralumenal content being dragged to the laparotomy entry site.

As an example of the latter approach, in U.S. Pat. No. 6,543,456 a method for gastric bypass surgery includes the insertion of proximal and distal anastomosis members (e.g., anvils) transorally with grasping forceps. The stomach and the small intestine are transected endoscopically by a surgical severing and stapling instrument to create a gastric pouch, a drainage loop, and a Roux limb. An endoscopically inserted circular stapler attaches to the distal anastomosis member to join the drainage loop to a distal portion of the intestine, and the circular stapler attaches to the proximal anastomosis member to join the Roux limb to the gastric pouch. Thereafter, the anastomosis members are removed to create an orifice between joined portions of the stomach and intestine. This method reduces the number of laparoscopic ports, avoids a laparoscopic insertion of an anastomosis instrument (e.g., circular stapler) into an enlarged surgical port, and eliminates the need for an enterotomy and an enterotomy closure.

For many anastomoses, surgeons use circular staplers, linear staplers, or manual sutures. However, to reduce incision size and to make the surgical process less technically demanding and time consuming, single piece anastomotic devices have been proposed which are deformable during deployment by an anastomotic applier to hold tissue portions together. US 2005/0070926 A1 and US 2005/0070935, describe an anastomotic applier having an implement portion that receives an anastomotic ring device with an unactuated shape of a cylinder with a proximal ring at one end and a distal ring at the other. The ring device further has proximal arms that are attached to the proximal ring and distal arms that are attached to the distal ring. Inwardly directed ends of the distal arms are coupled to inwardly directed ends of the proximal arms at a center ring such that the arms will outwardly actuate when the rings are drawn closer together during actuation of the applier. A latching mechanism comprises a hook protruding from the distal ring proximally such that it snap engages the proximal ring when the ring device assumes its actuated shape of a rivet. The applier comprises radially protruding catches which engage the ring such that the spacing of the distal ring to the center ring and the spacing of the proximal ring to the center ring may be reduced by a translational movement of the catches. In order to remove the applier from the actuated and latched ring device, proximal to each catch is a release ramp that causes the catch to move inwardly as the release ramp contacts the next more proximal ring at full actuation. The anastomotic ring device and applier disclosed in US 2005/0070926 A1 and US 2005/0070935 make it possible to perform a single lumen access anastomosis through existing trocar ports and to create an anastomotic attachment between lumens obviating the need for surgical stapling and suturing.

Nonetheless, this known devices have the disadvantage that the latching mechanism of the ring device locks the rings automatically when they reach their fully actuated configuration and this locking occurs at only one set configuration, i.e. ring distance, of the ring device. As a consequence, the ring device can be deployed and actuated with only one preset ring distance, without any possibility of ring distance adjustment. This means that different ring devices must be provided for different tissue thicknesses or different tissue compression rates and an adjustment of the tissue compression during deployment (i.e. adjustment of the distance between the distal and proximal rings) of the ring device is impossible.

Moreover, the automatic latching of the ring device might disturb the ring approximation and the correct positioning of the tissue walls between the rings during deployment and actuation of the anastomotic device.

Further examples of known anastomotic fastener means are described in EP 0517488 A1.

The object of the present invention is therefore to improve the known anastomotic ring device such that an adjustment of the ring compression or ring approximation (i.e. tissue thickness) becomes possible.

A further object of the present invention is to improve the known anastomotic ring device such that it doesn't lock automatically at a set actuated configuration. These and other objects are achieved by an anastomosis device according to the annexed claim 1. Advantageous embodiments are the object of the dependent claims. According to the invention, an anastomosis device comprises:
- a ring device having at least a proximal ring and a distal ring intended to be positioned on either side of a proximal and distal tissue portion and suitable to clamp the proximal and distal tissue portions between them when the ring device is actuated, wherein the rings define a central passage opening across the ring device (preferably the ring device comprise proximal, center, and distal rings connected respectively by proximal and distal hinged arms, the hinged arms having a generally oblong radially retracted shape when the ring device is unactuated and a folded radially protruding shape when the ring device is actuated, the proximal, center and distal rings defining a central passage opening across the ring device) ;
- a locking device adapted to lock the ring device when actuated,
wherein the locking device is separate from the ring device and insertable in the passage opening thereof and comprises:
- a first locking surface adapted to engage the distal ring and a second locking surface adapted to engage the proximal ring to latch the ring device;
- adjusting means for providing the first and second locking surfaces at different interjacent distances such as to latch the ring device in different actuated configurations said adjusting means comprising threaded means.

Thanks to the adjusting means the locking device can be adjusted to different distances between the distal and proximal rings, allowing the ring device to be deployed at different ring distances and different tissue pressures. Moreover, by providing a locking device separate from the ring device, the latter is not automatically latched and can be adjusted to the desired tissue compression prior to latching. As a result, the ring adjustment is not disturbed or influenced by the locking mechanism as happens with prior art devices and the same ring device and locking device can be utilized for different tissue thicknesses and tissue compression rates.

In accordance with an aspect of the invention, the adjusting means comprises threaded means interposed between the first and second locking surfaces such that the distance between the first and second surfaces is continuously or gradually adjustable by a rotational screw movement.

According to a preferred embodiment, the locking device comprises an annular proximal shoulder suitable to engage a proximal end surface of the proximal ring and a longitudinal portion which protrudes distally from the proximal shoulder and forms elastically supported snapper teeth extending radially outwardly from the longitudinal portion to enable snap engagement of the distal ring, wherein the annular proximal shoulder comprises a distally projecting externally threaded adjusting portion which mates an internal thread provided at the proximal base of the longitudinal portion such that the distance between the shoulder and the snapper teeth is adjustable by screwing the annular proximal shoulder more or less into the longitudinal portion.

In accordance with a further aspect of the invention, the adjusting means comprises two or more different first locking surfaces arranged in one or more rows extending in the longitudinal direction of the locking device such that each one of the different first locking surfaces of a same row have a different distance to the second locking surface. This makes it possible to lock the ring device at different discretely selectable ring distances by simply pushing the locking device from the proximal side into and across the ring device until the desired first locking surface engages the distal ring. Moreover, the longitudinal row/s of first locking surfaces allow to lock the ring device at intermediate ring distances in a way that the proximal and distal rings cannot be moved apart beyond this locked intermediate distance, while they can be further approximated. In order to lock the ring device in its final actuated configuration, the locking device can be advanced in a ratchet-like manner until the desired first locking surface engages the distal ring.

In accordance with a preferred embodiment, the locking device comprises an annular proximal shoulder suitable to engage a proximal end surface of the proximal ring and a longitudinal portion which protrudes distally from the proximal shoulder and forms elastically supported snapper teeth extending radially outwardly from the longitudinal portion to enable snap engagement of the distal ring, wherein the snapper teeth are arranged in a plurality of longitudinally extending rows such that each one of the different snapper teeth of a same row has a different distance to the proximal shoulder.

According to an advantageous embodiment, the elongate portion comprises a substantially cylindrical wall defining longitudinal window openings or slots and the snapper teeth are formed on radially elastically deflectable tongues arranged inside the window openings or slots of the wall. The tongues are preferably arranged at a constant angular pitch around the circumference of the cylindrical wall. This contributes to stabilize the anastomotic passage opening and keeps the ring device in a uniformly locked configuration.

In accordance with a further aspect of the invention, the cylindrical wall defines further longitudinal slots which enable the locking device to be pushed in engagement with the ring device without interfering with protruding actuating members of an anastomotic applier.

By providing the longitudinal slots, the locking device can be advantageously applied laparoscopically o endoscopically by means of an anastomotic applier which is also used to deploy and actuate the ring device.

In accordance with a yet further embodiment of the invention, the adjusting means comprises coarse adjustment means and additional fine adjustment means for adjusting the distance between the first and second locking surfaces, wherein the coarse adjustment means are preferably embodies by two or more different first locking surfaces arranged in one or more rows extending in the longitudinal direction of the locking device such that each one of the different first locking surfaces of a same row has a different discretely selectable distance to the second locking surface and the fine adjustment means comprises preferably threaded means interposed between the first and second locking surfaces such that the distance between the discretely selected first locking surface and the second locking surface can be continuously fine-adjusted by rotating the threaded means. This enables the surgeon to adjust very rapidly the distance at which the ring device is to be locked by simply ratchet like advancing the locking portion into and across the ring device and to perform a very precise fine adjustment of the ring distance by a screw movement of the threaded means prior or after locking the ring device.

According to an embodiment, also the coarse and fine adjustable locking device comprises (similar to the previously mentioned embodiments with only one adjusting feature) an annular proximal shoulder suitable to engage a proximal end surface of the proximal ring and a longitudinal portion which protrudes distally from the proximal shoulder and forms elastically supported snapper teeth extending radially outwardly from the longitudinal portion to enable snap engagement of the distal ring. The snapper teeth are arranged in a plurality of longitudinally extending rows such that each one of the different snapper teeth of a same row has a different distance to the proximal shoulder. Moreover, a threaded adjusting portion is provided which thread-connects the longitudinal portion with the annular proximal shoulder such that the longitudinal distance between the annular proximal shoulder and the snapper teeth is adjustable by screwing the adjusting portion with respect to the longitudinal portion.

These and other features and advantages of the present invention shall be made apparent from the accompanying drawings which illustrate embodiments of the invention, and, together with the general description of the invention given above, and the detailed description of the embodiments given below, serve to explain the principles of the present invention.
- FIG. 1 is a perspective view of a single lumen access deployable ring device for intralumenal anastomosis and a locking device not falling within the scope of the claims.
- FIG. 2 is a perspective view of the ring device and the locking device of figure 1, wherein the locking device is inserted in the ring device and locks the latter in an actuated configuration.
- FIG. 3 is a longitudinal sectional view of the ring device and locking device in figure 2.
- FIG. 4 is a perspective view of the ring device and a locking device according to a second embodiment of the invention.
- FIG. 5 is a perspective view of the ring device and the locking device of figure 4, wherein the locking device is inserted in the ring device and locks the latter in an actuated configuration.
- FIG. 6 is a longitudinal sectional view of the ring device and locking device in figure 5;
- FIG. 7 is a perspective view of the ring device and a locking device according to a third embodiment of the invention.
- FIG. 8 is a perspective view of the ring device and the locking device of figure 7, wherein the locking device is inserted in the ring device and locks the latter in an actuated configuration.
- FIG. 9 is a longitudinal sectional view of the ring device and locking device in figure 8.
- FIG. 10 is a perspective view of the ring device and the locking device installed upon an applier being inserted laparoscopically to an anastomosis target site on each of two portions of a patient's small intestine.
- FIG. 11 shows the applier, ring device and locking device of FIG. 10 after actuation and latching of the ring device to appose the two portions of small intestine.
- FIG. 12 to 15 are detail views of the applier, ring device and locking device in different operational configurations according to an embodiment of the invention.
- FIG. 16 and 17 are enlarged sectional views of an actuating portion of the applier in a lock position and release position, respectively.
- FIG. 18 is a schematic perspective view of a detail of the actuating mechanism of the applier according to an illustrative embodiment of the invention.

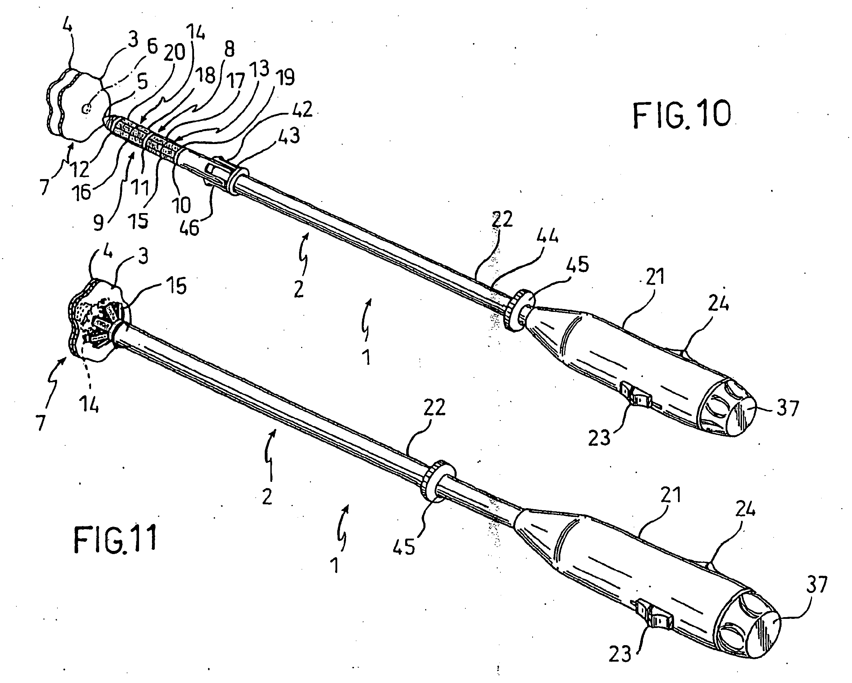

Turning to the Drawings, wherein like numerals denote like components throughout the several views, FIG. 10 depicts an applier 1 having an elongate implement portion 2 dimensionally sized for insertion through a cannula of a trocar or laparoscopic port to tissue walls 3, 4 to anastomose two lumens. A distal introducer tip 5 of the applier 1 pierces through an opening 6 at an anastomosis site 7 to position an actuating portion 8 that holds a ring device 9 for single lumen anastomosis and a locking device 42 for latching the ring device 9 in an actuated configuration.

The ring device 9 has three primary rings, depicted as a proximal ring 10, a center ring 11, and a distal ring 12, that are cylindrically aligned with one another. The proximal ring 10 is longitudinally attached to the center ring 11 by proximal arms 13, which in turn is longitudinally attached to the distal ring 12 by distal arms 14. Each proximal and distal arm 13, 14 is bisected respectively by a hinged joint 15, 16 defining an inner arm segment 17, 18 also hingedly attaching to the center ring 11 and an outer arm segment 19, 20 also hingedly attached to the respective proximal or distal ring 10, 12. In its unactuated state as depicted in FIG. 10, the arms 13, 14 are substantially oblong and the ring device 9 is at least approximately cylindrical. The relative lengths of the inner arm segments 17, 18 to outer arm segments 19, 20 may be selected to provide a desired angular contact to tissue walls 3, 4. In the illustrative version, the relationship resembles a cantilevered contact with the inner arm segments 17, 18 actuating to an approximately parallel relationship to the tissue walls 3, 4.

A handle portion 21 is proximally connected to a shaft 22 of the implement portion 2. The shaft 22 may be rigid or flexible, with the latter being desirable for intralumenal insertion, such as through the esophagus. The handle 21 includes controls for longitudinally positioning the rings 10, 11, 12 of the ring device 9 and for applying the locking device 42 to the ring device 9. In the illustrative version, the controls include a distal ring slide control 23, a proximal ring slide control 24 as well as a latching slide control 45. Although a manually positioned and actuated applier 1 is depicted for clarity, it should be appreciated that a remotely positioned and actuated applier may be used consistent with aspects of the invention, for instance to allow ring placement and latching in a more controlled manner, to avoid disturbing an imaging modality, or for other reasons. The handle 21 may further include controls for a distal tip illumination capability so that actuation of the distal arms 14 in the distal lumen may be proximally viewed from an endoscope. It will be appreciated that the terms "proximal" and "distal" are used herein with reference to a clinician gripping the handle portion 21 of the applier 1.

In FIG. 11, the distal ring slide control 23 has been withdrawn proximally and the proximal ring slide control 24 has been advanced distally so that both the distal ring 12 and proximal rings 10 are brought into locking proximity of the center ring 11, which remains substantially stationary. In response thereto, the proximal and distal arms 13, 14 hinge outwardly from the longitudinal axis of the ring device 9, creating a hollow rivet or hourglass shape for apposing tissue walls 3, 4. The center ring 11 sits at a tissue junction between lumens and the distal and proximal rings 12, 10 come to rest in respective lumens. By distally advancing the latching control 45, the locking device 42 has been advanced distally in snap engagement with rings 10, 12 so that the ring device 9 is held in the actuated position with bent arms 13, 14 apposing tissue. The proximal arms 13 may be staggered, as depicted, from distal arms 14 to create a tortuous path for the compressed tissue. Alternatively, the arms 13, 14 may be aligned to directly mate to each other.

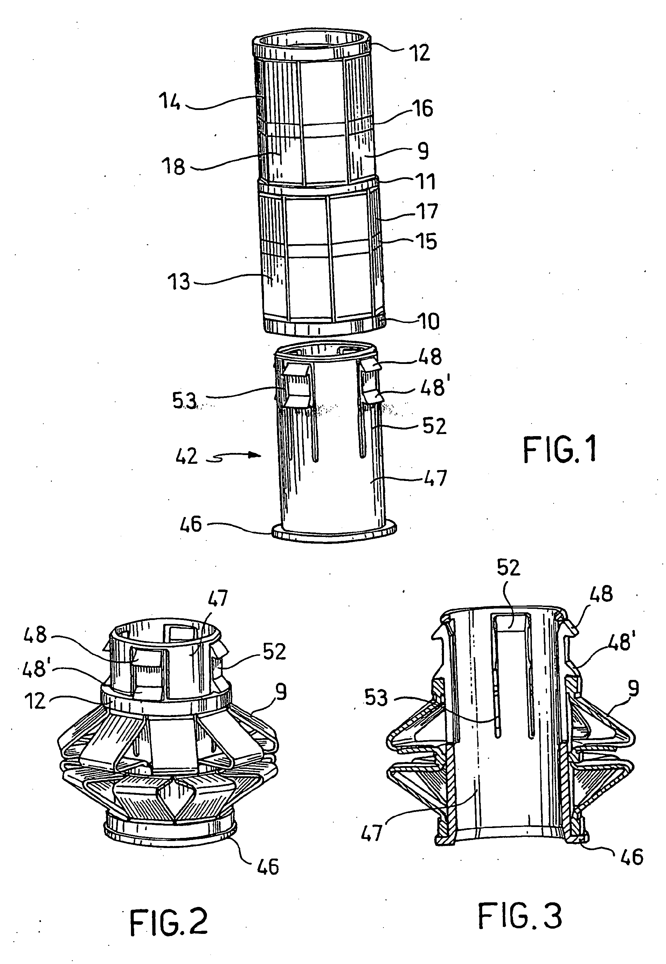

Turning now to the locking device, in accordance with a first embodiment not falling within the scope of the claims and illustrated in figures 1 to 3, the locking device 42 comprises an annular proximal shoulder 46 suitable to engage a proximal end surface of the proximal ring 10 and a longitudinal portion 47 which protrudes distally from the proximal shoulder 46 and forms elastically supported snapper teeth 48 extending radially outwardly from the longitudinal portion 47 to enable snap engagement of the distal ring 12.

According to the invention, the locking device 42 is adapted to latch the ring device 9 at different adjustable distances between the proximal ring 10 and the distal ring 12. This feature, together with a ring release mechanism of the applier (which will be described in detail below) suitable to release the ring device independently from its particular actuated configuration, make it possible to deploy the anastomotic ring device at different tissue thicknesses and pressure rates and to adjust the ring configuration and tissue pressure during the application of the ring device.

The longitudinal portion 47 comprises a substantially cylindrical wall defining longitudinal window openings or slots 53 and the snapper teeth 48, 48' are formed on radially elastically deflectable tongues 52 arranged inside said window openings or slots 53 in longitudinally extending rows such that each one of the (at least two) different snapper teeth 48, 48' of a same row (i.e. of the same tongue 52) has a different distance to said proximal shoulder 46 corresponding to different adjustable distances between the proximal and distal rings 10, 12 of the ring device 9.

In order to allow the locking device 42 to be applied by the same applier 1 which actuates also the ring device 9, the cylindrical wall defines further longitudinal slots 49 in alignment with the proximal catches or arresting surfaces and, preferably, also in alignment with the distal catches of the applier 1 which enable the locking device 42 to be pushed in engagement with the ring device 9 without interfering with these protruding catches of the applier (which will be described in detail below).

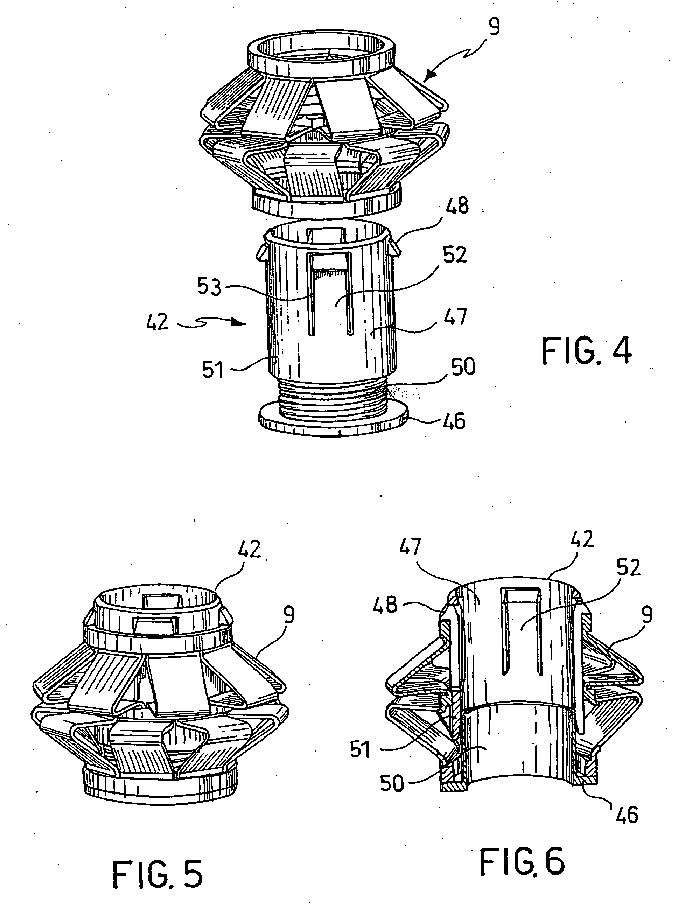

In accordance with a second embodiment (FIG. 4 to 6), the longitudinal portion 47 of the locking device 42 defines only one circumferential row of snapper teeth 48 (at least two diametrically oppositely arranged snapper teeth formed on two opposite tongues 52, respectively) suitable to snap engage the distal ring 12, but the annular proximal shoulder 46 comprises a distally projecting externally threaded adjusting portion 50 which mates an internal thread provided at the proximal base 51 of the longitudinal portion 47 such that the distance between the shoulder 46 and the snapper teeth 48 is adjustable by screwing the adjusting portion 50 more or less into the longitudinal portion 47. In order to adapt the locking device to the actuating mechanism of the applier, also the threaded adjusting portion 50 might be slotted in order not to interfere with the catches or arresting surfaces of the applier 1. In this particular embodiment, the screw adjustment must be performed in angular steps which correspond to the angular offset of the slots 49 or catches, respectively. Apart from the threaded adjusting portion 50 and the internal thread at the proximal base 51 of the longitudinal portion 47, the shape of the longitudinal portion 47 and the annular proximal shoulder 46 can be identical to those of the first embodiment.

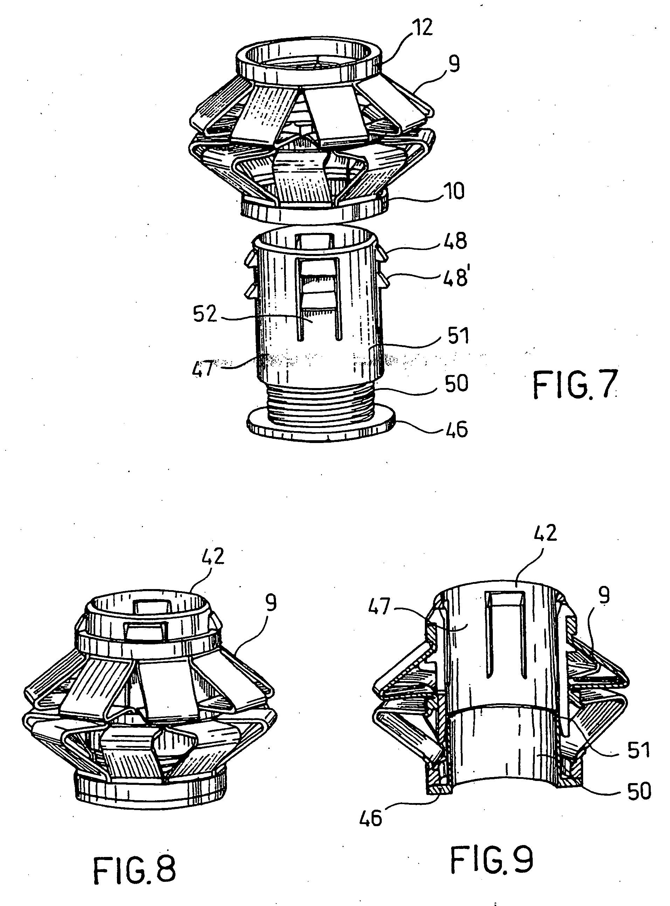

In accordance with a third embodiment (FIG. 7 to 9), the locking device 42 comprises coarse adjustment means and additional fine adjustment means. The coarse adjustment means are embodied by two or more snapper teeth 48, 48' arranged in each of a plurality of (preferably four) longitudinally extending rows such that the distances between the annular proximal shoulder 46 and the different snapper teeth 48, 48' of a same row correspond to different discretely adjustable distances between the proximal and distal rings 10, 12 of the ring device 9. The fine adjustment means are embodied by an externally threaded adjusting portion 50 which protrudes distally from the annular proximal shoulder 46 and mates an internal thread provided at the proximal base 51 of the longitudinal portion 47. In this way, the distance between the shoulder 46 and the discretely selected snapper tooth 48 is continuously fine adjustable by screwing the adjusting portion 50 more or less into the longitudinal portion 47.

Similar to the previously described embodiments, also in this embodiment the longitudinal portion 47 comprises preferably a substantially cylindrical wall defining longitudinal window openings or slots 53 and the snapper teeth 48 are formed on (preferably four equidistant) radially elastically deflectable tongues 52 arranged inside said window openings or slots 53. The cylindrical wall can be interrupted by further longitudinal slots 49 which enable the locking device 42 to be pushed in engagement with the ring device 9 without interfering with protruding actuating members of the anastomotic applier 1.

According to a yet further embodiment (not illustrated in the figures) the locking device 42 itself defines a seat for receiving a group of needles or staples such that, during application, the locking device 42 moves in snap engagement with the rings 10, 12 and pushes contemporaneously the group of needles or staples in the anastomotic ring device 9 thereby piercing the tissue 3, 4 held between the proximal and distal ring arms. In this case, the group of needles might advantageously comprise a self supporting needle ring which can be arranged on the annular shoulder 46 of the locking device 42.

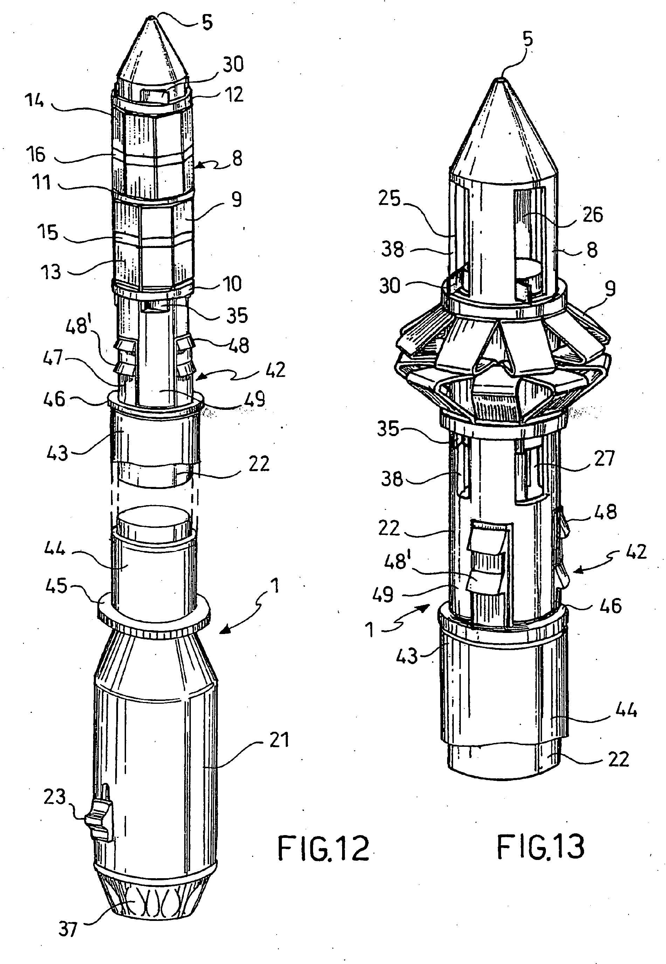

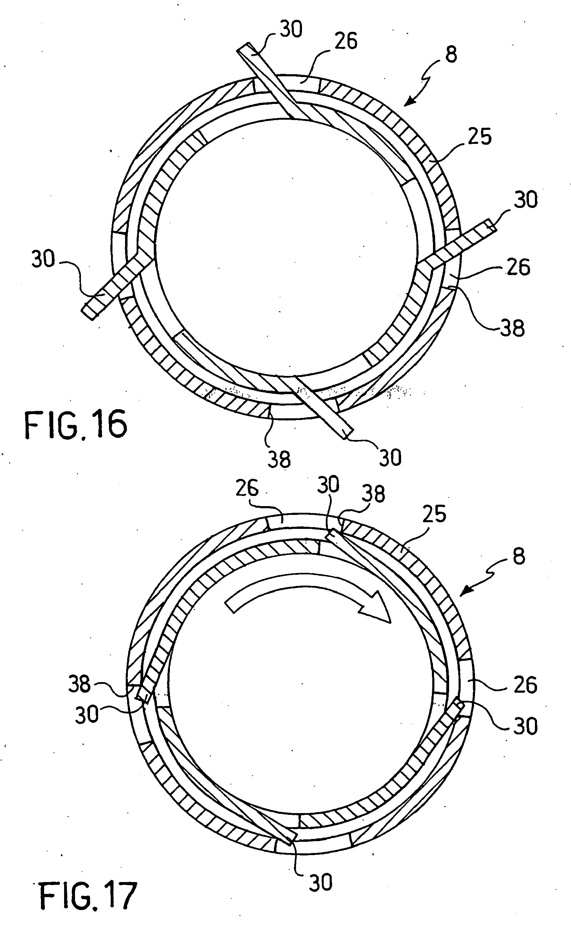

Figures 12 to 18 illustrate an embodiment of the applier 1 adapted to deploy and actuate the ring device 9 and to apply the locking device 42 to the actuated ring device 9 according to an embodiment of the invention.

The implement portion 2 comprises a substantially cylindrical housing 25 which defines, in the zone of the actuating portion 8, a plurality of axially extending distal window slots 26 and a plurality of axially extending proximal window slots 27 which extend along the track of the actuation movement of the distal and proximal rings 12, 10, respectively.

A distal actuating member 28 comprises a shaft 29 slidably received inside the housing 25 and operatively connected with the distal ring slide control 23 via a pull rod 31.

A plurality of fin shaped distal catches 30 protrude outward from the shaft 29 and extend through the distal window slots 26 such that they engage a distal end surface of the distal ring 12, when in the lock position. In this condition, it is possible to move the distal ring 12 proximally by shifting the distal ring slide control 23 in proximal direction.

Similarly, a proximal actuating member 32 comprises a shaft 33 slidably received inside the housing 25 and operatively connected with the proximal ring slide control 24 via a push rod 34. A plurality of fin shaped proximal catches 35 protrude outward from the shaft 33 and extend through the proximal window slots 27 such that they engage a proximal end surface of the proximal ring 10, when in the lock position. In this condition, it is possible to move the proximal ring 10 distally by shifting the proximal ring slide control 24 in distal direction.

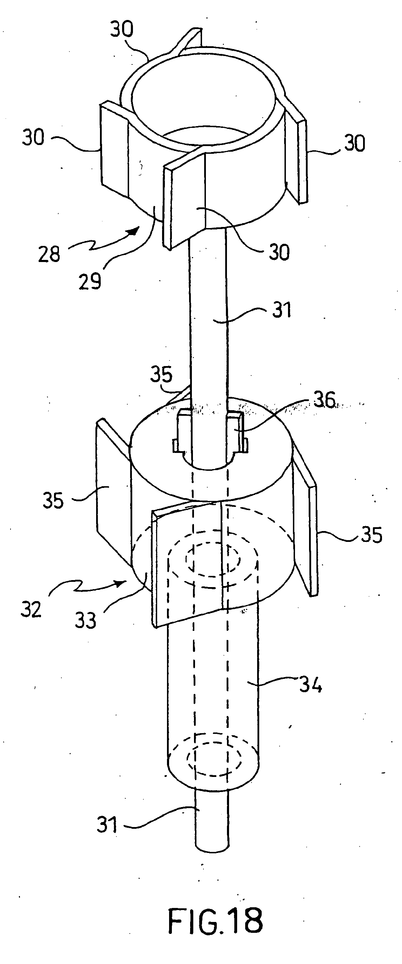

As schematically illustrated in figure 18, the proximal and distal actuating members 28, 32 are configured to translate relative to each other (and preferably independently from one another), but they are advantageously locked to each other in rotation about the longitudinal axis X of the implement portion 2, e.g. by a geometric coupling 36.

The handle 21 comprises a release control, e.g. a rotating knob 37, coupled to the pull rod 31 and/or to the push rod 34 and adapted to rotate at least the distal actuating member 28 (and preferably both actuating members 28, 32) about the longitudinal axis X of the implement portion 2 from the lock position to a release position in which the catches 30, 35 are disengaged from the anastomotic ring device 9 such that the implement portion of the applier can be proximally withdrawn through the center opening of the ring device 9.

The disengaging of the applier 1 from the ring device 9 is obtained by the feature that, during the rotation of the actuating member 28, 32, a part of the latter slides along a deviating surface at the implement portion, particularly at the housing 25, which is configured to urge the catches 30, 35 radially inward to disengage them at least from the distal ring 12. The skilled person will appreciate that, in order to withdraw the applier from the ring device, the proximal catches need not necessarily be retracted.

In accordance with the embodiment illustrated in the figures, the catches 30, 35 are preferably inclined or slanted in a direction opposite the direction of rotation from the lock position to the release position, thereby facilitating the inward deviation of the catches by lateral longitudinal edges 38 of the corresponding window slots 26, 27 which embody the above mentioned deviating surfaces.

In the illustrative version, the housing 25 and each actuating member 28, 32 is formed from a rigid polymer or sheet metal.

In accordance with the invention, the applier 1 comprises a latching mechanism adapted to carry the detachable locking device 42 and to apply it to the ring device 9 in its actuated rivet shape. The latching mechanism comprises one or more longitudinally translatable push portions 43 arranged proximally with respect to the proximal catches 35 or to stationary proximal arresting surfaces and adapted to engage a proximal end surface of the locking device 42 and to push the locking device 42 distally in engagement with the anastomotic ring device 9. The push portions 43 are connected, via a push rod (for instance a tubular push rod 44 externally applied to the implement portion 2) to the latching slide control, for instance a flange portion 45 of the push rod 44 arranged near the handle portion 21. This allows to latch the ring device 9 after its actuation, thereby obviating to automatic latching which might disturb fine adjustment of the ring approximation and of the compression rate applied to the tissue walls 3, 4 clamped by the ring device 9.

The flange portion 45 of the tubular push rod 44 apart from embodying a latching slide control (translational latching movement), preferably embodies also a rotational adjusting control for rotating the push portion 43 about the longitudinal axis of the implement portion 2. To this end, the push portion 43 is rotatably supported at the implement portion 2 and comprises preferably geometrical coupling means (e.g. teeth, recesses) suitable to engaging the annular shoulder 46 of the locking device 42 so that a rotational adjusting movement of the push portion 43 causes the annular shoulder 46 to screw into or unscrew from the longitudinal portion 47 which is preferably prevented to rotate, e.g. by the catches 35, 30 or by stationary arresting surfaces.

In FIG. 12, the oblong un-actuated ring device 9 is applied to the actuating portion 8 of the applier 1 and the distale 30 and proximal catches 35 protrude outward through the corresponding window slots 26, 27 and engage the distal and proximal rings 12, 10 of the ring device 9. The latching control 45 and the push portion 43 together with the locking device 42 are in a proximally retracted position.

FIG. 13 illustrates the situation after the distal ring slide control 23 has been shifted proximally and the proximal ring slide control 24 has been shifted distally, causing the actuating members 28, 32 to approximate the distal ring 12 and the proximal ring 10 toward the center ring 11 and the ring device 9 to deform in its actuated rivet shape. In this configuration the latching control 45 and the push portion 43 together with the locking device 42 are still proximally retracted and the distal and proximal catches 30, 35 of the applier 1 are still in their lock position, i.e. they protrude outward and inhibit withdrawal of the applier 1 from the actuated ring device 9.

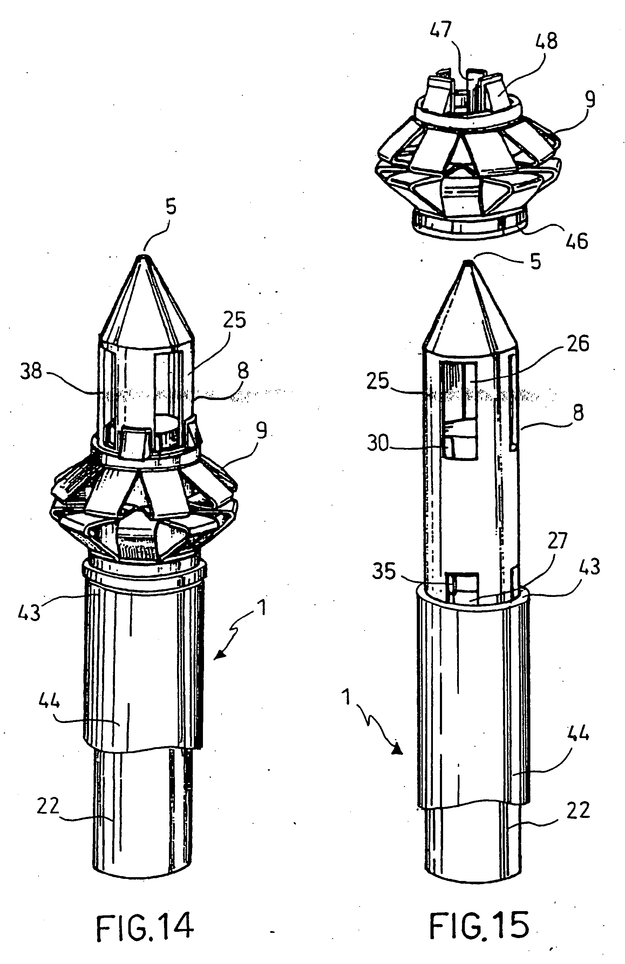

FIG. 14 illustrates the situation after the latching control 45 has been slid distally causing the locking device 42 to be pushed in snap engagement with the actuated ring device 9 which is effectively latched. After latching of the ring device, the release control 37 has been rotated, thereby rotating the actuating members 28, 32 about the longitudinal axis X from the lock position into the release position, in which the catches 30, 35 are bent inwardly by the window edges 38 and disengage from the ring device 9. It is now possible to remove the applier 1 proximally through the center opening of the actuated and deployed ring device 9, as shown in FIG. 15.

In use, the ring device 9 is received upon the actuating portion 8 of the implement portion 2 of the applier 1. Specifically, the proximal ring 10 of the ring device 9 rests against the proximal catches 35 and the distal catches 30 of the distal ring actuating member 28 engage the distal ring 12 of the ring device 9. A clinician manipulates the handle 21 to insert the implement portion 2 through the cannula of a trocar, laparoscopic port, or through a lumen such as the esophagus to the anastomosis site 7. The tissue walls 3, 4 are distally placed (from the surgeon point of view) and the introducer tip 5 of the implement portion 2 passes through the opening 6 formed in these walls 3, 4. The introducer tip may include a piercing shape and/or electromagnetically or thermally enhanced cutting features to assist in forming the opening 6. Once the distal arms 14 of the ring device 9 are in the distal lumen, the distal ring slide control 23 may be proximally moved to actuate the distal arms into a partially actuated ring shape. The distal tissue wall 4 thus held may be drawn back proximally if necessary such that the proximal arms 13 reside within the first lumen. Pushing the proximal ring slide control 24 gradually distally causes partial actuation of the proximal arms 13. If the positioning is correct, the slide controls 23, 24 may be fully slid, causing the proximal and distal arms 13, 14 to be fully actuated. By sliding the latching slide control 45 distally, the locking device 42 enters within the inner diameter of the ring device 9 and snap engages the distal and proximal rings thereof. It is now possible to disengage the catches 30, 35 that hold the applier 1 to the ring device 9 by rotating the release control 37 which causes the catches to retract radially. Then, the distal tip 5 of the applier is withdrawn from the ring device 9 leaving it deployed to form the anastomotic attachment. Over time, the tissue walls 3, 4 permanently heal together and the ring device 9 as well as the locking device 42 may be passed out of the digestive tract, especially if biofragmentable.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, aspects of the invention have application to surgical procedures performed endoscopically and laparoscopically, as well as an open procedure. Use herein of one of these or similar terms should not be construed to limit the present invention for use in only one category of surgical procedure.

Moreover, even though the figures illustrate an example of single double piece anastomotic ring device, it is not the intention of the inventors to limit the application of the locking device to the anastomotic device illustrated. As a matter of fact, the locking device is also suitable to be used in connection with double piece anastomotic ring devices of the type comprising two distinct compression rings which are intended to be arranged on either side of the two tissue walls which need to be joined in anastomosis. The compression rings are approximated and locked to one another in order to clamp the two tissue walls between them and the anastomotic orifice through the tissue walls is created by widening or resecting the tissue overlapping into an internal passage opening of the compression rings.

For another example, although bariatric procedures for bypassing portions of a gastrointestinal tract are depicted, it should be appreciated that other surgical procedures may benefit by a locking device having aspects described herein, such as for the bile duct and vascular bypasses.

## Claims

1. A locking device (42) for an anastomotic ring device (9) of the type comprising at least a proximal ring (10) and a distal ring (12) intended to be positioned on either side of a proximal and distal tissue portion and suitable to clamp the proximal and distal tissue portions between them when the ring device (9) is actuated, wherein the rings (10, 12) define a central passage opening across the ring device (9),
said locking device (42) being adapted to latch the ring device when actuated, wherein
the locking device (42) is separate from the ring device (9) and insertable in the passage opening thereof and comprises:
- a first locking surface (48, 48') adapted to engage the distal ring (12) and a second locking surface (46) adapted to engage the proximal ring (10) to lock the ring device (9);
**characterised in that** the locking device comprises:
- adjusting means (50, 51; 48, 48') for providing said first and second locking surfaces (46; 48, 48') at different interjacent distances such as to lock the ring device (9) in different actuated configurations
and **in that** said adjusting means comprises threaded means (50, 51) interposed between said first (48) and second (46) locking surfaces such that the distance between the first and second surfaces is rotatably adjustable.

2. A locking device (42) according to claim 1, comprising:
- an annular proximal shoulder (46) suitable to engage a proximal end surface of the proximal ring (10) and;
- a longitudinal portion (47) which protrudes distally from the proximal shoulder (46) and forms elastically supported snapper teeth (48) extending radially outwardly from the longitudinal portion (47) to enable snap engagement of the distal ring (12);
- a threaded adjusting portion (50) which thread-connects the longitudinal portion (47) with the annular proximal shoulder (46) such that the longitudinal distance between the annular proximal shoulder (46) and the snapper teeth (48) is adjustable by screwing the adjusting portion (50) with respect to the longitudinal portion (47).

3. A locking device (42) according to claim 2, wherein said annular proximal shoulder (46) comprises a distally projecting externally threaded adjusting portion (50) which mates an internal thread provided at a proximal base (51) of the longitudinal portion (47) such that the distance between the annular proximal shoulder (46) and the snapper teeth (48) is adjustable by screwing the annular proximal shoulder (46) more or less into the longitudinal portion (47).

4. A locking device (42) according to claim 3, wherein the longitudinal portion (47) comprises a substantially cylindrical wall defining longitudinal window openings or slots (53) and the snapper teeth (48) are formed on radially elastically deflectable tongues (52) arranged inside said window openings or slots (53).

5. A locking device (42) according to claim 4, wherein the cylindrical wall defines further longitudinal slots (49) which enable the locking device (42) to be pushed in engagement with the ring device (9) without interfering with protruding actuating members (30, 35) of an anastomotic applier (1).

6. A locking device (42) according to claim 5, in which also the threaded adjusting portion (50) is slotted in order to not interfere with the actuating members (35) of the anastomotic applier (1).

7. A locking device (42) according to claim 1, wherein said adjusting means comprises two or more different first locking surfaces (48, 48') arranged in one or more rows extending in the longitudinal direction of the locking device (42) such that each one of the different first locking surfaces (48, 48') of a same row has a different distance to said second locking surface (46).

8. A locking device (42) according to claim 7, comprising:
- an annular proximal shoulder (46) suitable to engage a proximal end surface of the proximal ring (10) and;
- a longitudinal portion (47) which protrudes distally from the proximal shoulder (46) and forms elastically supported snapper teeth (48, 48') extending radially outwardly from the longitudinal portion (47) to enable snap engagement of the distal ring (12),
wherein said snapper teeth (48, 48') are arranged in a plurality of longitudinally extending rows such that each one of the different snapper teeth (48, 48') of a same row has a different distance to said proximal shoulder (46).

9. A locking device (42) according to claim 8, wherein the longitudinal portion (47) comprises a substantially cylindrical wall defining longitudinal window openings or slots (53) and the snapper teeth (48, 48') are forged on radially elastically deflectable tongues (52) arranged inside said window openings or slots

10. A locking device (42) according to claim 9, wherein the cylindrical wall defines further longitudinal slots (49) which enable the locking device (42) to be pushed in engagement with the ring device (9) without interfering with protruding actuating members (30, 35) of an anastomotic applier (1).

11. A locking device (42) according to claim 1, wherein said adjusting means comprises coarse adjustment means (48, 48') and fine adjustment means (50, 51) for adjusting the distance between the first and second locking surfaces (46; 48, 48'), wherein said fine adjustment means comprises the threaded means (50, 51).

12. A locking device (42) according to claim 11, wherein said coarse adjustment means (48, 48') comprises two or more different first locking surfaces (48, 48') arranged in one or more rows extending in the longitudinal direction of the locking device (42) such that each one of the different first locking surfaces (48, 48') of a same row has a different distance to said second locking surface (46), which can be discretely selected, and said fine adjustment means (50, 51) comprises said threaded means (50) interposed between said first and second locking surfaces such that the distance between the discretely selected first locking surface (48, 48') and said second locking surface (46) can be continuously adjusted by rotating the threaded means (50).

13. A locking device (42) according to claim 12, comprising:
- an annular proximal shoulder (46) suitable to engage a proximal end surface of the proximal ring (10) and;
- a longitudinal portion (47) which protrudes distally from the proximal shoulder (46) and forms elastically supported snapper teeth (48, 48') extending radially outwardly from the longitudinal portion (47) to enable snap engagement of the distal ring (12),
wherein said snapper teeth (48, 48') are arranged in a plurality of longitudinally extending rows such that each one of the different snapper teeth of a same row has a different distance to said proximal shoulder (46).
- a threaded adjusting portion (50) which thread-connects the longitudinal portion (47) with the annular proximal shoulder (46) such that the longitudinal distance between the annular proximal shoulder (46) and the snapper teeth (48, 48') is adjustable by screwing the adjusting portion (50) with respect to the longitudinal portion (47).

14. An anastomosis device comprising:
- a ring device (9) having proximal (10), center (11), and distal (12) rings connected respectively by proximal and distal hinged arms (13, 14), the hinged arms (13, 14) having a generally oblong radially retracted shape when the ring device (9) is unactuated and a folded radially protruding shape when the ring device (9) is actuated, said proximal, center and distal rings defining a central passage opening across the ring device (9); !
- a locking device (42) according to any one of the preceding claims.

## Patentansprüche

1. Verriegelungsvorrichtung (42) für eine Anastomose-Ringvorrichtung (9) von der Art, welche wenigstens einen proximalen Ring (10) und einen distalen Ring (12) umfasst, welche bestimmt sind, um auf beiden Seiten eines proximalen und distalen Gewebeabschnitts positioniert zu werden, und geeignet sind, den proximalen und distalen Gewebeabschnitt zwischen sich zu klemmen, wenn die Ringvorrichtung (9) betätigt wird, wobei die Ringe (10, 12) eine zentrale Passagenöffnung durch die Ringvorrichtung (9) definieren, wobei die Verriegelungsvorrichtung (42) geeignet ist, die Ringvorrichtung zu verrasten, wenn sie betätigt wird, wobei die Verriegelungsvorrichtung (42) von der Ringvorrichtung (9) separat und in die Passagenöffnung davon einfügbar ist und eine erste Verriegelungsfläche (48, 48'), die geeignet ist, mit dem distalen Ring (12) in Eingriff zu stehen, und eine zweite Verriegelungsfläche (46) umfasst, die geeignet ist, mit dem proximalen Ring (10) in Eingriff zu stehen, um die Ringvorrichtung (9) zu verriegeln;
**dadurch gekennzeichnet, dass** die Verriegelungsvorrichtung Einstellmittel (50, 51; 48, 48') umfasst, zum Bereitstellen der ersten und zweiten Verriegelungsflächen (46; 48, 48') an verschiedenen, dazwischenliegenden Abständen, um die Ringvorrichtung (9) in verschiedenen betätigten Konfigurationen zu verriegeln,
und dass die Einstellmittel Gewindemittel (50, 51) umfassen, welche zwischen ersten (48) und zweiten (46) Verriegelungsflächen angeordnet sind, so dass der Abstand zwischen den ersten und zweiten Flächen drehbar einstellbar ist.

2. Verriegelungsvorrichtung (42) nach Anspruch 1, umfassend:
- eine ringförmige, proximale Schulter (46), die geeignet ist, mit einer proximalen Endfläche des proximalen Rings (10) in Eingriff zu stehen, und
- einen länglichen Abschnitt (47), welcher distal von der proximalen Schulter (46) vorsteht und elastisch gelagerte Schnappzähne (48) bildet, welche sich radial nach außen von dem länglichen Abschnitt (47) erstrecken, um einen Schnappeingriff des distalen Rings (12) zu ermöglichen;
- einen Gewindeeinstellabschnitt (50), welcher den länglichen Abschnitt (47) mit der ringförmigen, proximalen Schulter (46) gewindeverbindet, so dass der längliche Abstand zwischen der ringförmigen, proximalen Schulter (46) und den Schnappzähnen (48) durch Schrauben des Einstellabschnitts (50) bezüglich des länglichen Abschnitts (47) einstellbar ist.

3. Verriegelungsvorrichtung (42) nach Anspruch 2, wobei die ringförmige, proximale Schulter (46) einen distal vorstehenden Außengewindeeinstellabschnitt (50) umfasst, welcher mit einem Innengewinde ineinander greift, welches an einem proximalen Fuß (51) des länglichen Abschnitts (47) bereitstellt ist, so dass der Abstand zwischen der ringförmigen, proximalen Schulter (46) und den Schnappzähnen (48) durch Schrauben der ringförmigen, proximalen Schulter (46) mehr oder weniger in den länglichen Abschnitt (47) einstellbar ist.

4. Verriegelungsvorrichtung (42) nach Anspruch 3, wobei der längliche Abschnitt (47) eine im Wesentlichen zylindrische Wandung umfasst, die längliche Fensteröffnungen oder Schlitze (53) definiert, und wobei die Schnappzähne (48) an radial elastisch ablenkbaren Zungen (52) gebildet sind, welche innerhalb der Fensteröffnungen oder Schlitze (53) angeordnet sind.

5. Verriegelungsvorrichtung (42) nach Anspruch 4, wobei die zylindrische Wandung ferner längliche Schlitze (49) definiert, welche es ermöglichen, dass die Verriegelungsvorrichtung (42) in Eingriff mit der Ringvorrichtung (9) gedrückt wird, ohne vorstehende Betätigungselemente (30, 35) eines Anastomose-Applikator (1) zu beeinträchtigen.

6. Verriegelungsvorrichtung (42) nach Anspruch 5, wobei auch der Gewindeeinstellabschnitt (50) geschlitzt ist, um nicht die Betätigungselemente (35) des Anastomose-Applikator (1) zu beeinträchtigen.

7. Verriegelungsvorrichtung (42) nach Anspruch 1, wobei die Einstellmittel zwei oder mehr verschiedene erste Verriegelungsflächen (48, 48') umfassen, welche in einer oder mehreren Reihen angeordnet sind, die sich in die Längsrichtung der Verriegelungsvorrichtung (42) erstrecken, so dass jede der verschiedenen ersten Verriegelungsflächen (48, 48') einer gleichen Reihe einen unterschiedlichen Abstand zu der zweiten Verriegelungsfläche (46) aufweist.

8. Verriegelungsvorrichtung (42) nach Anspruch 7, umfassend:
- eine ringförmige, proximale Schulter (46), die geeignet ist, mit einer proximalen Endfläche des proximalen Rings (10) in Eingriff zu sein, und
- einen länglichen Abschnitt (47), welcher distal von der proximalen Schulter (46) vorsteht und elastisch gelagerte Schnappzähne (48, 48') bildet, welche sich radial nach außen von dem länglichen Abschnitt (47) erstrecken, um einen Schnappeingriff des distalen Rings (12) zu ermöglichen,
wobei die Schnappzähne (48, 48') in einer Mehrzahl von sich länglich erstreckenden Reihen angeordnet sind, so dass jeder der verschiedenen Schnappzähne (48, 48') von einer gleichen Reihe einen unterschiedlichen Abstand zu der proximalen Schulter (46) aufweist.

9. Verriegelungsvorrichtung (42) nach Anspruch 8, wobei der längliche Abschnitt (47) eine im Wesentlichen zylindrische Wandung umfasst, welche längliche Fensteröffnungen oder Schlitze (53) definiert, und wobei die Schnappzähne (48, 48') an radial elastisch ablenkbaren Zungen (52) gebildet sind, welche innerhalb der Fensteröffnungen oder Schlitze (53) angeordnet sind.

10. Verriegelungsvorrichtung (42) nach Anspruch 9, wobei die zylindrische Wandung ferner längliche Schlitze (49) definiert, welche es ermöglichen, dass die Verriegelungsvorrichtung (42) in Eingriff mit der Ringvorrichtung (9) gedrückt wird, ohne vorstehende Betätigungselemente (30, 35) eines Anastomose-Applikator (1) zu beeinträchtigen.

11. Verriegelungsvorrichtung (42) nach Anspruch 1, wobei die Einstellmittel Grobeinstellmittel (48, 48') und Feineinstellmittel (50, 51) zum Einstellen des Abstandes zwischen den ersten und zweiten Verriegelungsflächen (46; 48, 48') umfassen, wobei die Feineinstellmittel die Gewindemittel (50, 51) umfassen.

12. Verriegelungsvorrichtung (42) nach Anspruch 11, wobei die Grobeinstellmittel (48, 48') zwei oder mehr verschiedene erste Verriegelungsflächen (48, 48') umfassen, welche in einer oder mehreren Reihen angeordnet sind, die sich in die Längsrichtung der Verriegelungsvorrichtung (42) erstrecken, so dass jede der verschiedenen ersten Verriegelungsflächen (48, 48') einer gleichen Reihe einen unterschiedlichen Abstand zu der zweiten Verriegelungsfläche (46) aufweist, welche diskret ausgewählt werden können, und das die Feineinstellmittel (50, 51) die Gewindemittel (50) umfassen, welche zwischen den ersten und zweiten Verriegelungsflächen angeordnet sind, so dass der Abstand zwischen der diskret gewählten ersten Verriegelungsfläche (48, 48') und der zweiten Verriegelungsfläche (46) kontinuierlich durch Drehen der Gewindemittel (50) eingestellt werden kann.

13. Verriegelungsvorrichtung (42) nach Anspruch 12, umfassend:
- eine ringförmige, proximale Schulter (46), die geeignet ist, mit einer proximalen Endfläche des proximalen Rings (10) in Eingriff zu sein, und
- einen länglichen Abschnitt (47), welcher distal von der proximalen Schulter (46) vorsteht und elastisch gelagerte Schnappzähne (48, 48') bildet, welche sich radial nach außen von dem länglichen Abschnitt (47) erstrecken, um einen Schnappeingriff des distalen Rings (12) zu ermöglichen,
wobei die Schnappzähne (48, 48') in einer Mehrzahl von sich länglich erstreckenden Reihen angeordnet sind, so dass jeder der verschiedenen Schnappzähne von einer gleichen Reihe einen unterschiedlichen Abstand zu der proximalen Schulter (46) aufweist,
- einen Gewindeeinstellabschnitt (50), welcher den länglichen Abschnitt (47) mit der ringförmigen, proximalen Schulter (46) gewindeverbindet, so dass der längliche Abstand zwischen der ringförmigen, proximalen Schulter (46) und den Schnappzähnen (48, 48') durch Schrauben des Einstellabschnitts (50) bezüglich des länglichen Abschnitts (47) einstellbar ist.

14. Eine Anastomose-Vorrichtung, umfassend:
- eine Ringvorrichtung (9) mit proximalen (10), zentralen (11) und distalen (12) Ringen die jeweils durch proximale und distale schwenkbare Arme (13, 14) verbunden sind, wobei die schwenkbaren Arme (13, 14) eine gemeinhin längliche radial eingezogene Form aufweisen, wenn die Ringvorrichtung (9) nicht betätigt ist, und eine gefaltete radial vorstehende Form, wenn die Ringvorrichtung (9) betätigt ist, wobei die proximalen, zentralen und distalen Ringe eine zentrale Passagenöffnung über die Ringvorrichtung (9) definieren;
- eine Verriegelungsvorrichtung (42) nach einem der vorhergehenden Ansprüche.

## Revendications

1. Dispositif de verrouillage (42) pour un dispositif formant bague d'anastomose (9) du type comprenant au moins une bague proximale (10) et une bague distale (12) destinées à être positionnées de chaque côté de parties de tissu proximale et distale et appropriées pour serrer les parties de tissu proximale et distale entre elles lorsque le dispositif formant bague (9) est actionné, dans lequel les bagues (10, 12) définissent une ouverture de passage centrale à travers le dispositif formant bague (9),
ledit dispositif de verrouillage (42) étant adapté pour verrouiller le dispositif formant bague lorsqu'il est actionné, dans lequel
le dispositif de verrouillage (42) est séparé du dispositif formant bague (9) et peut être inséré dans l'ouverture de passage de celui-ci et comprend :
- une première surface de verrouillage (48, 48') adaptée pour venir en prise avec la bague distale (12) et une deuxième surface de verrouillage (46) adaptée pour venir en prise avec la bague proximale (10) pour verrouiller le dispositif formant bague (9) ;
**caractérisé en ce que** le dispositif de verrouillage comprend :
- des moyens d'ajustement (50, 51 ; 48, 48') pour placer lesdites première et deuxième surfaces de verrouillage (46 ; 48, 48') à différentes distances les unes par rapport aux autres de manière à verrouiller le dispositif formant bague (9) dans différentes configurations d'actionnement,
et **en ce que** lesdits moyens d'ajustement comprennent des moyens filetés (50, 51) interposés entre lesdites première (48) et deuxième (46) surfaces de verrouillage de sorte que la distance entre les première et deuxième surfaces puisse être ajustée par rotation.

2. Dispositif de verrouillage (42) selon la revendication 1, comprenant :
- un épaulement proximal (46) annulaire approprié pour venir en prise avec une surface d'extrémité proximale de la bague proximale (10) ; et
- une partie longitudinale (47) qui fait saillie distalement de l'épaulement proximal (46) et qui forme des dents d'encliquetage (48) supportées de manière élastique s'étendant radialement vers l'extérieur de la partie longitudinale (47) pour permettre une mise en prise par encliquetage de la bague distale (12) ;
- une partie d'ajustement (50) filetée qui relie par vissage la partie longitudinale (47) à l'épaulement proximal (46) annulaire de sorte que la distance longitudinale entre l'épaulement proximal (46) annulaire et les dents d'encliquetage (48) puisse être ajustée en vissant la partie d'ajustement (50) par rapport à la partie longitudinale (47).

3. Dispositif de verrouillage (42) selon la revendication 2, dans lequel ledit épaulement proximal (46) annulaire comprend une partie d'ajustement (50) filetée extérieurement faisant saillie distalement qui s'accouple à un filetage interne prévu au niveau d'une base proximale (51) de la partie longitudinale (47) de sorte que la distance entre l'épaulement proximal (46) annulaire et les dents d'encliquetage (48) puisse être ajustée en vissant l'épaulement proximal (46) annulaire plus ou moins dans la partie longitudinale (47).

4. Dispositif de verrouillage (42) selon la revendication 3, dans lequel la partie longitudinale (47) comprend une paroi sensiblement cylindrique définissant des ouvertures de fenêtre ou fentes (53) longitudinales et les dents d'encliquetage (48) sont formées sur des languettes (52) pouvant être défléchies radialement de manière élastique agencées à l'intérieur desdites ouvertures de fenêtre ou fentes (53).

5. Dispositif de verrouillage (42) selon la revendication 4, dans lequel la paroi cylindrique définit en outre des fentes (49) longitudinales qui permettent que le dispositif de verrouillage (42) soit poussé en prise avec le dispositif formant bague (9) sans interférer avec des éléments d'actionnement (30, 35) saillants d'un applicateur d'anastomose (1).

6. Dispositif de verrouillage (42) selon la revendication 5, dans lequel la partie d'ajustement (50) filetée est également fendue afin de ne pas interférer avec les éléments d'actionnement (35) de l'applicateur d'anastomose (1).

7. Dispositif de verrouillage (42) selon la revendication 1, dans lequel lesdits moyens d'ajustement comprennent deux premières surfaces de verrouillage (48, 48') différentes ou plus agencées en une ou plusieurs rangées s'étendant dans la direction longitudinale du dispositif de verrouillage (42) de sorte que chacune des différentes premières surfaces de verrouillage (48, 48') d'une même rangée soit à une distance différente de ladite deuxième surface de verrouillage (46).

8. Dispositif de verrouillage (42) selon la revendication 7, comprenant :
- un épaulement proximal (46) annulaire approprié pour venir en prise avec une surface d'extrémité proximale de la bague proximale (10) ; et
- une partie longitudinale (47) qui fait saillie distalement de l'épaulement proximal (46) et qui forme des dents d'encliquetage (48, 48') supportées de manière élastique s'étendant radialement vers l'extérieur de la partie longitudinale (47) pour permettre une mise en prise par encliquetage de la bague distale (12),
dans lequel lesdites dents d'encliquetage (48, 48') sont agencées en une pluralité de rangées s'étendant longitudinalement de sorte que chacune des différentes dents d'encliquetage (48, 48') d'une même rangée soit à une distance différente dudit épaulement proximal (46).

9. Dispositif de verrouillage (42) selon la revendication 8, dans lequel la partie longitudinale (47) comprend une paroi sensiblement cylindrique définissant des ouvertures de fenêtre ou fentes (53) longitudinales et les dents d'encliquetage (48, 48') sont formées sur des languettes (52) pouvant être défléchies radialement de manière élastique agencées à l'intérieur desdites ouvertures de fenêtre ou fentes (53).

10. Dispositif de verrouillage (42) selon la revendication 9, dans lequel la paroi cylindrique définit en outre des fentes (49) longitudinales qui permettent que le dispositif de verrouillage (42) soit poussé en prise avec le dispositif formant bague (9) sans interférer avec les éléments d'actionnement (30, 35) saillants d'un applicateur d'anastomose (1).

11. Dispositif de verrouillage (42) selon la revendication 1, dans lequel lesdits moyens d'ajustement comprennent des moyens d'ajustement grossier (48, 48') et des moyens d'ajustement fin (50, 51) pour ajuster la distance entre les première et deuxième surfaces de verrouillage (46 ; 48, 48'), dans lequel lesdits moyens d'ajustement fin comprennent les moyens filetés (50, 51).

12. Dispositif de verrouillage (42) selon la revendication 11, dans lequel lesdits moyens d'ajustement grossier (48, 48') comprennent deux premières surfaces de verrouillage (48, 48') différentes ou plus agencées en une ou plusieurs rangées s'étendant dans la direction longitudinale du dispositif de verrouillage (42) de sorte que chacune des différentes premières surfaces de verrouillage (48, 48') d'une même rangée soit à une distance différente de ladite deuxième surface de verrouillage (46), qui peut être sélectionnée de manière discrète, et lesdits moyens d'ajustement fin (50, 51) comprennent lesdits moyens filetés (50) interposés entre lesdites première et deuxième surfaces de verrouillage de sorte que la distance entre la première surface de verrouillage (48, 48') sélectionnée de manière discrète et ladite deuxième surface de verrouillage (46) puisse être ajustée en continu en tournant les moyens filetés (50).

13. Dispositif de verrouillage (42) selon la revendication 12, comprenant :
- un épaulement proximal (46) annulaire approprié pour venir en prise avec une surface d'extrémité proximale de la bague proximale (10) ; et
- une partie longitudinale (47) qui fait saillie distalement de l'épaulement proximal (46) et qui forme des dents d'encliquetage (48, 48') supportées de manière élastique s'étendant radialement vers l'extérieur de la partie longitudinale (47) pour permettre une mise en prise par encliquetage de la bague distale (12),
dans lequel lesdites dents d'encliquetage (48, 48') sont agencées en une pluralité de rangées s'étendant longitudinalement de sorte que chacune des différentes dents d'encliquetage d'une même rangée soit à une distance différente dudit épaulement proximal (46),
- une partie d'ajustement (50) filetée qui relie par vissage la partie longitudinale (47) à l'épaulement proximal (46) annulaire de sorte que la distance longitudinale entre l'épaulement proximal (46) annulaire et les dents d'encliquetage (48, 48') puisse être ajustée en vissant la partie d'ajustement (50) par rapport à la partie longitudinale (47).

14. Dispositif d'anastomose comprenant :
- un dispositif formant bague (9) comportant des bagues proximale (10), centrale (11) et distale (12) respectivement reliées par des bras (13, 14) articulés proximaux et distaux, les bras (13, 14) articulés ayant une forme radialement rétractée généralement oblongue lorsque le dispositif formant bague (9) n'est pas actionné et une forme faisant saillie radialement pliée lorsque le dispositif formant bague (9) est actionné, lesdites bagues proximale, centrale et distale définissant une ouverture de passage centrale à travers le dispositif formant bague (9) ;
- un dispositif de verrouillage (42) selon l'une quelconque des revendications précédentes.
